# EUROPÄISCHE PATENTSCHRIFT

(11) **EP 2 056 727 B1**
(45) Veröffentlichungstag und Bekanntmachung des Hinweises auf die Patenterteilung: **19.10.2011**
(21) Anmeldenummer: 07801841.3
(22) Anmeldetag: 23.08.2007
(51) Int. Cl.: A61B 17/28

(54) **CHIRURGISCHE GREIFZANGE**
SURGICAL GRIPPING FORCEPS
PINCE CHIRURGICALE

(30) Priorität: 30.08.2006 DE 102006040529
(43) Veröffentlichungstag der Anmeldung: 13.05.2009
(73) Patentinhaber: Paul Peschke Gmbh, 88637 Leibertingen (DE)
(72) Erfinder: REINAUER, Josef, 72488 Sigmaringen (DE); GANTER, Hans, 78532 Tuttlingen (DE)
(74) Vertreter: Patentanwälte Westphal, Mussgnug & Partner
(86) Internationale Anmeldenummer: PCT/EP2007/007412
(87) Internationale Veröffentlichungsnummer: WO 2008/025480

(56) Entgegenhaltungen:
- EP-A- 1 543 786
- WO-A-02/45599
- WO-A-98/33436
- WO-A-02/064020
- DE-A1- 10 056 238
- DE-A1- 10 316 132

## Beschreibung

Die Erfindung betrifft eine chirurgische Greifzange mit zwei gegenüber einem Grundkörper beweglichen Greifbacken, wobei jede Greifbacke eine gegenüber dem Grundkörper ortsfeste Schwenkachse und einen Hebelarm hat und wobei die Hebelarme über mindestens ein Schubelement angelenkt sind.

Aus der US 5,342,390 ist eine derartige Greifzange bekannt. Die Greifbacken dieser Zange sind auf einem gemeinsam genutzten Schwenkzapfen gelagert. Der Schwenkzapfen schneidet die Mittellinie des Zangengrundkörpers. Dadurch können die an den Greifbacken angeformten Hebelarme, über die Greifbacken bewegt werden, zwangsläufig nur relativ kurz ausgebildet werden. Auch wirkt auf jeden Hebelarm der Greifbacken jeweils ein separates Schubelement.

Der vorliegenden Erfindung liegt die Aufgabe zugrunde, eine chirurgische Greifzange zu entwickeln, die bei einer üblichen Zangenbetätigungskraft zwischen den Greifbacken eine große Zangenklemmkraft ermöglicht. Hierbei soll bei kleiner Zangenbaugröße eine geringe Bauteileanzahl realisiert werden.

Diese Aufgabe wird mit den Merkmalen des Anspruchs 1 gelöst. Dazu hat zumindest eine, vorzugsweise jede Greifbacke eine eigene Schwenkachse. Die einzelne Schwenkachse hat von der Mittellinie des Grundkörpers mindestens einen Abstand, der größer als 38% der maximalen Grundkörperbreite oder des maximalen Grundkörperdurchmessers ist.

Die laparoskopische Chirurgie benötigt ein spezielles Instrumentarium. Allen Instrumenten gemeinsam ist die Miniaturisierung, weshalb die laparoskopische Chirurgie auch endoskopische Mikrochirurgie genannt wird. Die Instrumente werden durch lange Hülsen, die einen Durchmesser haben, der in der Regel zwischen vier bis zwölf Millimetern liegt, in das Abdomen meist über Torkarhülsen eingeführt und außerhalb der Bauchhöhle mit Handkraft bedient.

In der Mikrochirurgie müssen sowohl feine Greifinstrumente für die Präparation als auch grobe für die Extraktion resezierter Organe vorhanden sein. Verschiedene Greifinstrumente mit einem Durchmesser von drei, fünf oder zehn Millimetern stehen zur Verfügung. Es gibt auf der einen Seite atraumatische und auf der anderen gezahnte Pinzetten. Sie haben Greifbacken, die spitz oder breit, fein oder grob sind. Teilweise verfügen die Greifinstrumente auch über Arretiermechanismen.

Die Greifzangen müssen einfach und sicher zu bedienen sein. Dazu gehört u.a. eine große Handkraftübersetzung durch den Greifzangenmechanismus. Besonders vorteilhaft ist es auch, wenn die Greifzange, also der Teil, der im Abdomen vorn aus der Torkarhülse herausschaut, möglichst wenige Teile hat. Wenige Teile haben immer auch wenige Bewegungsfugen. Dies senkt das Verletzungsrisiko und erleichtert das Desinfizieren der Greifzange. Letzteres gilt nur, sofern es sich bei der entsprechenden Zange nicht um ein Einweggerät handelt.

Weitere Einzelheiten der Erfindung ergeben sich aus den Unteransprüchen und der nachfolgenden Beschreibung einer schematisch dargestellten Ausführungsform:
- Figur 1:: Greifzange, geschlossen; in dimetrischer Ansicht;
- Figur 2:: Greifzange, geöffnet; von vorn dargestellt;
- Figur 3:: wie Figur 2, jedoch von hinten dargestellt;
- Figur 4:: Greifzange, geschlossen; in der Draufsicht;
- Figur 5:: Prinzipskizze zu halber Greifzange, geschlossen;
- Figur 6:: wie Figur 5, jedoch offen;
- Figur 7:: Prinzipskizze zu ganzer Greifzange, geschlossen;
- Figur 8:: wie Figur 7, jedoch offen;
- Figur 9:: Greifzange mit nur einer Greifbacke, geschlossen;
- Figur 10:: wie Figur 9, jedoch halb offen;
- Figur 11:: wie Figur 9, jedoch ganz offen;
- Figur 12:: Grundkörper in dimetrischer Ansicht;
- Figur 13:: Schubelement in dimetrischer Ansicht;
- Figur 14:: Greifbacke in dimetrischer Ansicht;
- Figur 15:: Greifzange weiterer Ausführungsform, geöffnet; von hinten dargestellt;
- Figur 16:: Greifbacke gemäß Fig. 15 in dimetrischer Ansicht; von unten dargestellt;
- Figur 17:: Schubelement gemäß Fig. 15 in dimetrischer Ansicht.

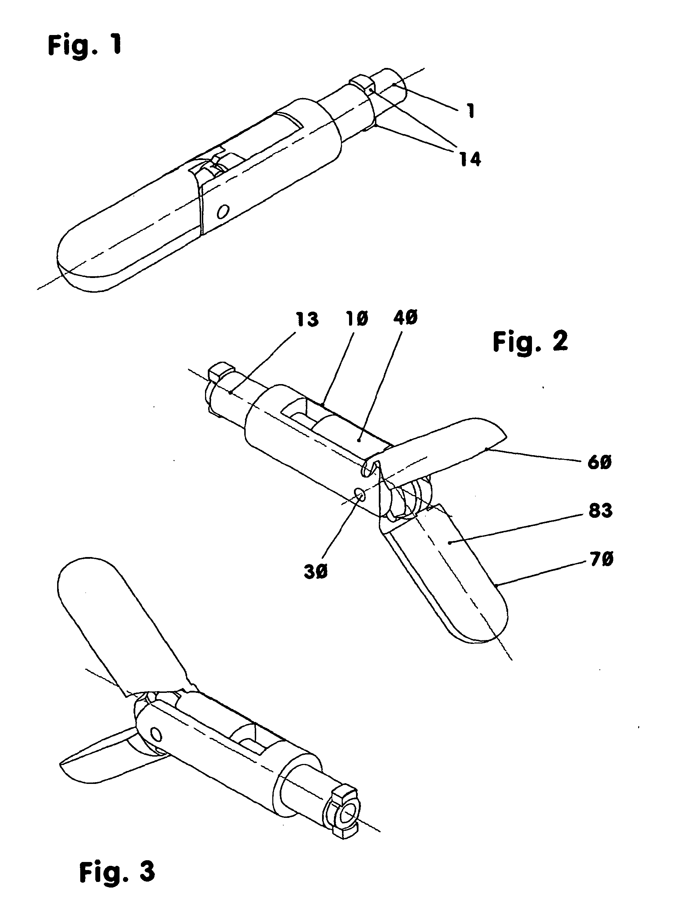

Die Figuren 1 - 3 zeigen eine chirurgische Greifzange in geschlossener und geöffneter Darstellung. Die Greifzange umfasst einen Grundkörper 10, ein Schubelement 40, zwei Greifbacken 60, 70 und einen Führungsbolzen 30.

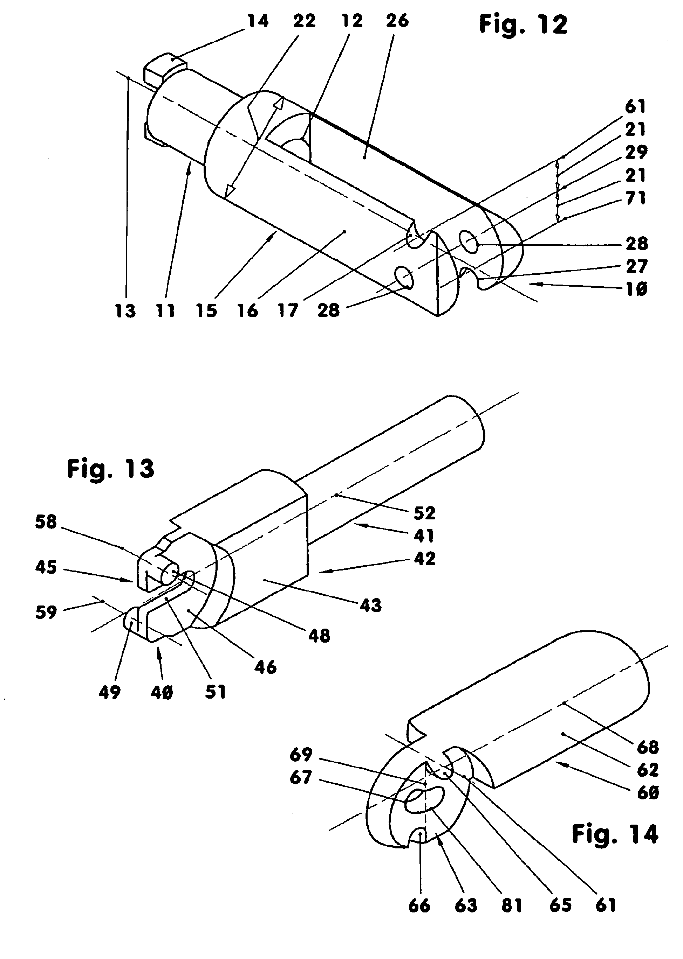

Der Grundkörper 10, vgl. auch Figur 12, besteht aus einem Rohr- 11 und einem Gabelabschnitt 15. Der Rohrabschnitt 11 hat eine zentrale Bohrung 12, in der das Schubelement 40 geführt ist. An seinem hinteren Ende sind zwei einander gegenüberliegende Adapterelemente 14 angeformt. Über diese Adapterelemente 14 wird der Grundkörper 10 an einem nicht dargestellten Gehäuserohr lösbar, z.B. mittels eines Bajonettverschlusses, befestigt.

Der Gabelabschnitt 15 hat zwei Gabelarme 16, 26, die am Rohrabschnitt 11 angeordnet sind. Die Außenwandung der Gabelarme 16, 26 sind beispielsweise Teile eines Zylindermantels. Der Durchmesser dieses Zylinders ist der Grundkörperdurchmesser 22. Er beträgt beim Ausführungsbeispiel 4,8 mm. Die Innenwandungen der Gabelarme 16, 26 sind Ebenen, die einander parallel gegenüber liegen. Der Abstand der Ebenen entspricht z.B. dem Innendurchmesser der zentralen Bohrung 12. An ihren vorderen Enden haben die Gabelarme 16, 26 jeweils eine Bohrung 28. Die zueinander fluchtenden Bohrungen 28 haben eine Mittellinie 29, die sich mit der Mittellinie 13 des Grundkörpers 10 senkrecht schneidet.

Der nach Figur 12 vordere Gabelarm 16 hat oberhalb der Bohrung 28 eine z.B. u-förmige, nutartige Ausnehmung 17. Der Ausnehmungsgrund hat bereichsweise die Fläche eines Zylindermantels. Das Zentrum des Zylindermantels ist eine obere Schwenkachse 61. Der hintere Gabelarm 26 hat eine vergleichbare Ausnehmung 27. Letztere ist hier nach unten orientiert und umgibt bereichsweise eine untere Schwenkachse 71. Die Schwenkachsen 61, 71 und die Mittellinie 29 der Bohrungen 28 liegen in einer Ebene. Diese Ebene ist normal zur Mittellinie 13 des Grundkörpers 10 ausgerichtet.

Figur 13 zeigt das Schubelement 40. Es besteht aus einem Schubzapfenabschnitt 41, einem Führungsabschnitt 42 und einem Lagerabschnitt 45. Der Schubzapfenabschnitt 41, über den das Schubelement 40 im Grundkörper 10 gelagert ist, hat eine zylindrische Form. An seinem freien Ende trägt er ggf. eine Gewindebohrung. In letzterer wird dann die im Gehäuserohr der Zange geführte Betätigungsstange lösbar befestigt. Die Gewindebohrung, das Gehäuserohr und die Betätigungsstange sind in den Figuren nicht dargestellt.

An den Schubzapfenabschnitt 41 schließt sich der Führungsabschnitt 42 an. Letzterer hat zumindest annähernd die Form eines Quaders mit zwei planen, zueinander parallelen Seitenflächen 43. Diese Seitenflächen 43 liegen bei der montierten Greifzange an den Innenwandungen der Gabelarme 16, 26 des Grundkörpers 10 an. Dort haben sie u.a. die Funktion einer Verdrehsicherung. Die oben und unten an die Seitenflächen 43 angrenzenden gekrümmten Zylinderteilflächen gehören zu einem Zylinder, dessen Durchmesser dem Grundkörperdurchmesser entspricht.

Der Führungsabschnitt 42 geht in einen Lagerabschnitt 45 über. Der Lagerabschnitt 45 entspricht einer dünnwandigen Platte, die zwei Gelenkzapfen 48, 49 und eine Führungsnut 51 aufweist. Die Gelenkzapfen 48, 49 haben zueinander parallele Mittellinien 58, 59. Beide Mittellinien 58, 59 spannen eine Ebene auf, die normal zur Mittellinie 52 des Schubelements 40 liegt. Der nach Figur 13 obere Gelenkzapfen 48 ist nach vorn orientiert, während der untere 49 nach hinten ausgerichtet ist. Beide Gelenkzapfen 48, 49 haben gegenüber der Mittellinie 52 den gleichen Abstand. Der Abstand zwischen den Mittellinien 58, 59 beträgt mehr als 2/3 des Grundkörperdurchmessers 22. Die Gelenkzapfen 48, 49 haben z.B. einen Durchmesser von 1 mm.

Mittig zwischen den Gelenkzapfen 48, 49 befindet sich eine gerade Führungsnut 51, die zum freien Ende des Lagerabschnitts 45 hin offen ist. Das geschlossene Ende der Führungsnut 51 hat eine teilzylindrische Ausrundung. Die Mittellinie der Ausrundung schneidet die Mittellinie 52 des Bauteils 40 senkrecht.

In Figur 14 ist eine von zwei Greifbacken 60 dargestellt. Im räumlichen Verhältnis zu den in den Figur 12 und 13 dargestellten Bauteilen 10 und 40 ist es die obere Greifbacke. Die Greifbacke 60 besteht aus einem Backenabschnitt 62 und einem Schwenkbereichsabschnitt 63. Der Backenabschnitt 62 hat die Form der Hälfte eines längsgeteilten Zylinders. Der Durchmesser dieses Zylinders stimmt mit dem Grundkörperdurchmesser 22 überein. Das vordere freie Ende des Backenabschnitts 62 ist abgerundet. Der Abrundungsradius entspricht dem halben Grundkörperdurchmesser 22.

Der zumindest annähernd kreisscheibenförmige Schwenkbereichsabschnitt 63 erstreckt sich in der vorderen Hälfte der Greifbacke 60. D.h. die hintere plane Fläche des Schwenkbereichsabschnitts 63 - sie kontaktiert eine Lagerfläche 46 des Lagerabschnitts 45 des Schubelements 40 - liegt in einer Ebene, die von der Bauteilmittenebene um die halbe Breite des Lagerabschnitts 45 entfernt ist. In dieser Mittenebene verläuft die Bauteilmittellinie 68. Auf letzterer liegt auch die hier ebene, atraumatische Backengreiffläche 83, vgl. Figur 2.

Im oberen Bereich hat der Schwenkbereichsabschnitt 63 einen sich nach vorn erstreckenden Schwenkzapfen 65. Der Schwenkzapfen 65, der eine Mittellinie 61 aufweist, hat zumindest nach unten hin eine zylindrische Außenkontur. Unterhalb des Schwenkzapfens 65 befindet sich eine sichelförmige Führungsausnehmung 67. Der Krümmungsradius der Führungsausnehmung 67 hat einen Mittelpunkt, der auf der Mittellinie 61 des Schwenkzapfens 65 liegt. Folglich verfügt die Führungsausnehmung 67 über mindestens eine kreisbogenförmige Kante 81, deren Mittelpunkt ebenfalls auf der Mittellinie 61 liegt. Unterhalb der Führungsausnehmung 67 ist in den Schwenkbereichsabschnitt 63 von unten her eine Kulissenausnehmung 66 eingearbeitet. Die Kulissenausnehmung 66 ist beispielsweise eine gerade Nut, deren Nutbreite geringfügig größer ist als der Durchmesser der Gelenkzapfen 48, 49 des Schubelements 40. Der Ausnehmungsgrund hat auch hier bereichsweise die Fläche eines Zylindermantels.

In Figur 14 ist auf der sichtbaren planen Fläche, die bei montierter Greifzange an der Innenwandung des entsprechenden Gabelarms 16, 26 des Grundkörpers 10 anliegt, eine gestrichelte Hilfslinie 69 eingezeichnet. Die Hilfslinie 69 steht zudem senkrecht auf der Ebene der Backengreiffläche 83. An dieser Hilfslinie 69 liegen die vorderste Mantellinie des Schwenkzapfens 65 und die Kante der hinteren, ebenen Wandung der Kulissenausnehmung 66 an.

Das fünfte und letzte Bauteil der Greifzange ist der zylindrische Führungsbolzen 30, vgl. Figur 2. Sein Durchmesser beträgt beim Ausführungsbeispiel 1 mm. Seine Länge ist geringfügig kleiner als der Grundkörperdurchmesser 22. Er sitzt in den Bohrungen 28 der Greifarme 16, 26 beispielsweise mittels eines Querpresssitzes.

Alle fünf Teile 10, 30, 40, 60, 70 der Greifzange sind beispielsweise jeweils aus einem rost- und säurebeständigen Stahl, z.B. dem Chromstahl X20Crl3 gefertigt.

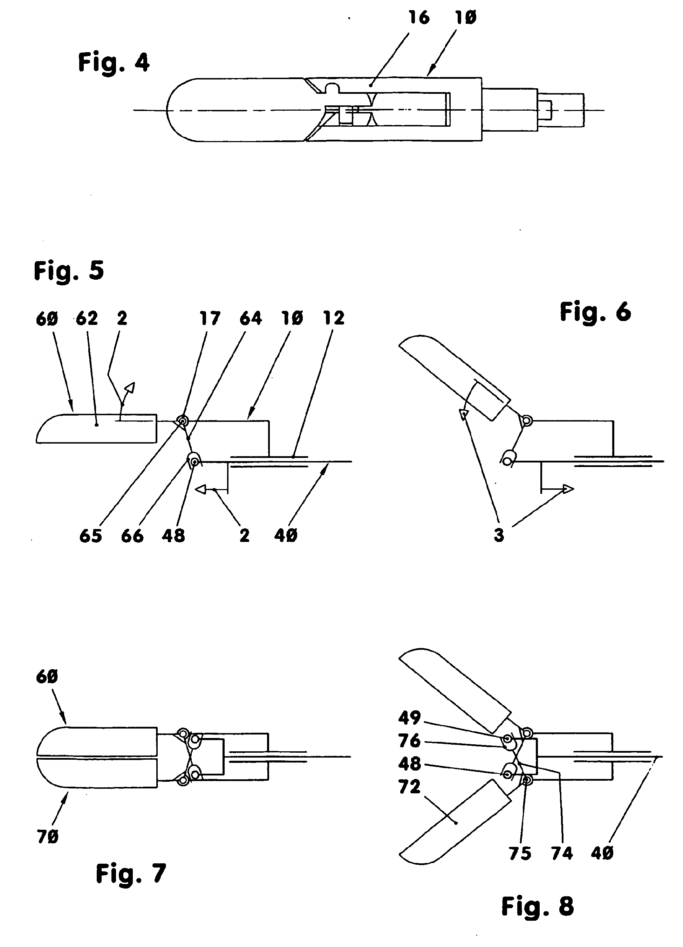

Bevor das Zusammenwirken der Zangenteile erläutert wird, wird kurz auf das Funktionsprinzip eingegangen. In den Figuren 5 und 6 ist das Funktionsprinzip zunächst für nur eine Greifbacke 60 dargestellt.

Nach Figur 5 ist die Greifbacke 60 ein Backenabschnitt 62, an dem ein Hebelarm 64 mit einer Kulissenausnehmung 66 und einem Schwenkzapfen 65 befestigt ist. Der Grundkörper 10 ist eine Geradführung 12 mit einem daran angeordneten Schwenkzapfenlager 17. In der Geradführung 12 ist das Schubelement 40 gelagert. Es greift über einen Gelenkzapfen 48 in die Kulissenausnehmung 66 ein.

Zum Öffnen des Greifbackens 60 wird das Schubelement 40 in die Geradführung 12 nach links verschoben. Der Gelenkzapfen 48 wirkt auf die Kulissenführung 66. Diese schwenkt zusammen mit dem Hebelarm 64 und dem Backenabschnitt 62 im Uhrzeigersinn nach oben. Hierbei dreht sich der Schwenkzapfen 65 im Schwenkzapfenlager 17 des Grundkörpers 10, vgl. Figur 6.

In den Figuren 7 und 8 ist das Prinzip für die gesamte Greifzange dargestellt. Dazu werden die in den Figuren 5 und 6 dargestellten Skizzen zunächst nach unten gespiegelt. Anschließend werden Original und Spiegelbild solange ineinandergeschoben, bis nach Figur 4 die Greifbacken 60, 70 aneinander liegen. Um nun hier mit einer Geradführung 12 auszukommen, werden beide Gelenkzapfen 48, 49 auf einem gemeinsamen Schubelement 40 fest angeordnet.

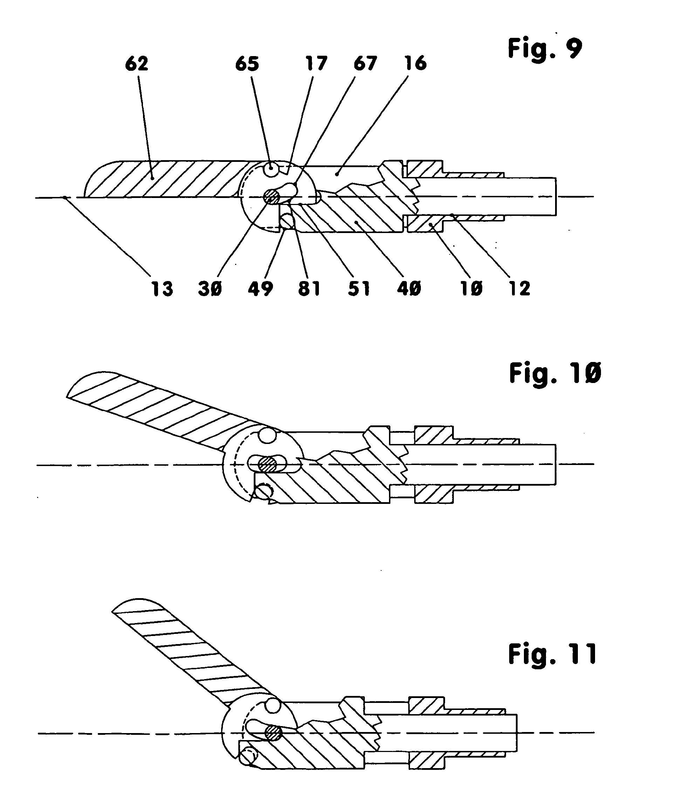

Die Einzelteile 10, 30, 40, 60, 70 der realen Greifzange, vgl. Figuren 12 bis 14, werden montiert, indem das Schubelement 40 mit seinem Führungsabschnitt 42 voraus fast vollständig in die Bohrung 12 des Grundkörpers 10 geschoben wird. Die Einstecktiefe ist in Figur 9 dargestellt. Nun wird die obere Greifbacke 60 von oben her in den Spalt zwischen dem vorderen Gabelarm 16 und der Lagerfläche 46 des Schubelements 40 eingelegt. Die Greifbacke 60 ist.hierbei in Strecklage, d.h. ihre Mittellinie 68 verläuft während des Einsteckvorganges parallel zur Mittellinie 13 des Grundkörpers 10. Bei der Abwärtsbewegung kommt der Schwenkzapfen 65 der Greifbacke 60 in der Ausnehmung 17 des Grundkörpers 10 zur Anlage. Zeitgleich wird die Kulissenausnehmung 66 der Greifbacke 60 über den Gelenkzapfen 59 des Schubelements 40 geschoben. Das Einlegen der Greifbacke 60 ist beendet, sobald sich die Mittellinie 68 der Greifbacke 60 mit der Mittellinie 13 des Grundkörpers 10 deckt. Die untere Greifbacke 70 wird in vergleichbarer Weise von unten her eingelegt.

Bei dieser Bauteilanordnung liegen im Gelenkbereich der Greifzange fünf Ausnehmungen bzw. Bohrungen zumindest bereichsweise übereinander. Dies sind - von außen nach innen betrachtet - die beiden Bohrungen 28 der Gabelarme 16, 26, die beiden sichelförmigen Führungsausnehmungen 67, 77 der Greifbacken 60, 70 und die Führungsnut 51 des Schubelements 40. Durch alle Ausnehmungen hindurch wird abschließend der Führungsbolzen 30 gesteckt und fixiert, vgl. Figuren 1 bis 4.

Die bisher nicht beschriebene Funktion der sichelförmigen Ausnehmung 67, der Greifbacke 60 wird in den Figuren 9 bis 11 verdeutlicht. In diesen Figuren ist die Greifzange im Längsschnitt ohne die untere Greifbacke 70 dargestellt. Aus dem Schubelement 40 ist zudem ein Stück ausgebrochen.

Der Grundkörper 10 lagert die Greifbacke 60 in der Ausnehmung 17 und auf dem Führungsbolzen 30. In der einseitig offenen Ausnehmung 17 liegt der Schwenkzapfen 65. Die sichelförmige Führungsausnehmung 67 umgreift den Führungsbolzen 30. Um nun ein unbeabsichtigtes Auswandern des Schwenkzapfens 65 bzw. der Greifbacke 67 nach oben zu verhindern, liegt die Führungsausnehmung 66 an dem Führungsbolzen 30 zumindest über die Kante 81 an. Durch die Anlage der Führungsausnehmung 67 am Führungsbolzen 30 ist es möglich, die Schwenkachse 61 der Greifbacke'60 innerhalb des Grundkörpers 10 weit von der Mittellinie 13 entfernt zu legen. Dies erlaubt einen großen Greifzangenhebelarm.

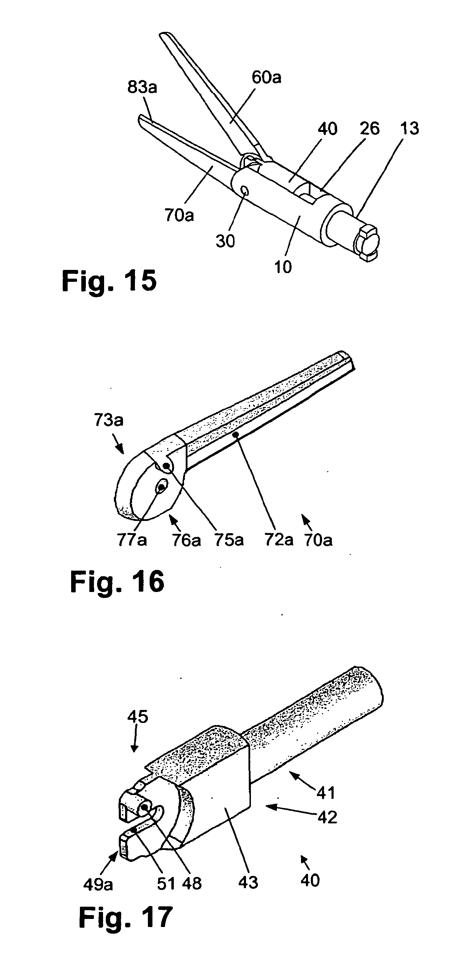

Die Figuren 15 - 17 zeigen eine chirurgische Greifzange gemäß einer modifizierten weiteren Ausführungsform in geöffneter Darstellung. Bezüglich dieser Ausführungsform werden unter Verweis auf die Fig. 1 - 14 und deren Beschreibung im Wesentlichen nur demgegenüber abweichende Merkmale beschrieben. In den Figuren kennzeichnen entsprechend gleiche Bezugszeichen gleiche oder gleich wirkende Elemente und mit einem "a" gekennzeichnete Bezugszeichen abweichende Elemente.

Die Greifzange umfasst ebenfalls einen Grundkörper 10, ein Schubelement 40, zwei Greifbacken 60a, 70a und einen Führungsbolzen 30. Dabei ist jedoch nur die eine der Greifbacken 60a mittels eines Führungsbolzens 30 im Grundkörper 10 beweglich gelagert. Die andere der Greifbacken 70a ist hingegen feststehend im Grundkörper 10 eingesetzt oder einteilig mit diesem ausgebildet.

In Figur 16 ist die nicht bewegliche der Greifbacken 70a dargestellt. Im räumlichen Verhältnis zu den in den Figur 15 und 17 dargestellten Bauteilen 10 und 40 ist es die untere Greifbacke. Die nicht bewegliche Greifbacke 70a besteht ebenfalls aus einem Backenabschnitt 72a hat jedoch anstelle eines Schwenkbereichsabschnitts einen modifizierten rückwärtigen Abschnitt 73a. Der Backenabschnitt 62 hat bei dieser Ausführungsform z.B. die Form eines länglichen Stegs oder einer verbreiterten Schneide.

Der rückwärtige Abschnitt 73a erstreckt sich wie bei der ersten Ausführungsform in der vorderen Hälfte der Greifbacke 70a. D.h. die hintere plane Fläche des rückwärtigen Abschnitts 73a kontaktiert eine Lagerfläche des Lagerabschnitts des Schubelements 40 und liegt in einer Ebene, die von der Bauteilmittenebene insbesondere um die halbe Breite des Lagerabschnitts entfernt ist. In dieser Mittenebene verläuft die Bauteilmittellinie. Auf letzterer liegt auch die hier z.B. ebenfalls ebene, atraumatische Backengreiffläche 83a (Figur 15).

Im oberen Bereich hat der rückwärtige Abschnitt 73a einen sich nach vorn erstreckenden Zapfen 75a in Art eines Schwenkzapfens der ersten Ausführungsform. Der Zapfen 75a, der eine Mittellinie 58 aufweist, hat zumindest nach unten hin eine zylindrische Außenkontur. Unterhalb des Zapfens 75a befindet sich anstelle einer sichelförmigen Führungsausnehmung eine zylindrische Ausnehmung 77a. Die zylindrische Ausnehmung 77a sitzt auf dem Führungsbolzen 30 (Fig. 15). Durch diese Ausgestaltung ist diese untere Greifbacke 70a in dem Grundkörper 10 nicht beweglich bzw. nicht verschwenkbar eingesetzt.

Alternativ könnte entsprechend auch ein Grundkörper 10 eingesetzt werden, der einteilig mit einer solchen Greifbacke 70a ausgebildet ist.

Figur 17 zeigt ein modifiziertes Schubelement 40. Es besteht wieder aus einem Schubzapfenabschnitt 41, einem Führungsabschnitt 42 und einem Lagerabschnitt. An den Schubzapfenabschnitt 41 schließt sich der Führungsabschnitt 42 an. Letzterer hat wieder z.B. die Form eines Quaders mit zwei planen, zueinander parallelen Seitenflächen 43. Der Führungsabschnitt 42 geht in einen vorderseitigen Lagerabschnitt 45 über.

Der Lagerabschnitt 45 entspricht wieder z.B. einer dünnwandigen Platte, die bei dieser Ausführungsform eine Führungsnut 51 aber hier nur einen derartigen Gelenkzapfen 48 aufweist. Mit anderen Worten fehlt auf der gegenüberliegenden Seite ein Gelenkzapfen 49a, welche zum Betätigen der feststehenden Greifbacke 70a dienen würde. In der Greifbacke 70a ist im Gegensatz zu der entsprechenden Greifbacke in dem rückwärtigen Abschnitt 73a unterhalb der Ausnehmung 77a entsprechend keine Kulissenausnehmung 76a von unten her erforderlich.

### Bezugszeichenliste

- 1: Längsachse bzw. Mittellinie der Greifzange
- 2: Öffnungsrichtung
- 3: Schließrichtung

- 10: Grundkörper
- 11: Rohrabschnitt
- 12: Bohrung, zentral; Geradführung
- 13: Mittellinie von (10)
- 14: Adapterelemente
- 15: Gabelabschnitt
- 16: Gabelarm
- 17: Ausnehmung, Schwenkzapfenlager

- 21: Schwenkachsenabstand
- 22: Grundkörperdurchmesser

- 26: Gabelarm
- 27: Ausnehmung, Schwenkzapfenlager
- 28: Bohrungen für Führungsbolzen
- 29: Mittellinie

- 30: Führungsbolzen

- 40: Schubelement
- 41: Schubzapfenabschnitt
- 42: Führungsabschnitt
- 43: Seitenflächen, plan
- 45: Lagerabschnitt
- 46: Lagerflächen
- 48: Gelenkzapfen
- 49: Gelenkzapfen
- 49a: fehlender Gelenkzapfen bei weiterer Ausführungsform

- 51: Führungsnut, gerade
- 52: Mittellinie zu (40)
- 58: Mittellinie zu (48)
- 59: Mittellinie zu (49)

- 60: erste Greifbacke
- 60a: erste Greifbacke weiterer Ausführungsform
- 61: Schwenkachse

- 62: Backenabschnitt
- 63: Schwenkbereichsabschnitt
- 64: Hebelarm
- 65: Schwenkzapfen
- 66: Kulissenausnehmung
- 67: Führungsausnehmung, sichelförmig
- 68: Mittellinie
- 69: Hilfslinie

- 70: zweite Greifbacke
- 70a: zweite Greifbacke weiterer Ausführungsform
- 71: Schwenkachse
- 72: Backenabschnitt
- 72a: Backenabschnitt weiterer Ausführungsform
- 73a: rückseitiger Abschnitt
- 74: Hebelarm
- 75: Schwenkzapfen
- 75a: Zapfen weiterer Ausführungsform
- 76: Kulissenausnehmung
- 76a: fehlende Kulissenausnehmung bei weiterer Ausführungsform
- 77: Führungsausnehmung, sichelförmig
- 77a: Ausnehmung weiterer Ausführungsform, zylindrisch

- 81: Kante
- 83: Backengreiffläche
- 83a: Backengreiffläche weiterer Ausführungsform

## Patentansprüche

1. Chirurgische Greifzange mit zwei Greifbacken (60, 70; 60a, 70a), wobei gegenüber einem Grundkörper (10) zumindest eine der Greifbacken (60) beweglich angeordnet sind, wobei die bewegliche der Greifbacken (60; 60a) eine gegenüber dem Grundkörper (10) ortsfeste Schwenkachse und einen Hebelarm (64) hat und wobei der Hebelarm (64) über mindestens ein Schubelement (40) angelenkt ist, wobei die bewegliche Greifbacken (60) eine eigene Schwenkachse (61; 62) hat, wobei die Schwenkachse (61) von der Mittellinie (13) des Grundkörpers (10) mindestens einen Abstand (21) hat, der größer als 38% der maximalen Grundkörperbreite oder des maximalen Grundkörperdurchmessers (22) ist
**dadurch gekennzeichnet,**
- **dass** die Schwenkachse (61) der einen beweglichen Greifbacke (60) durch einen greifbackenseitigen Schwenkzapfen (65, 75) und eine grundkörpereoitige Ausnehmung (17, 27) gebildet ist, und
- **dass** die einzelne bewegliche bzw. der beiden beweglichen Greifbacke (60, 70) in der Nähe des Schwenkzapfens (65, 75) eine Führungsausnehmung (67, 77) aufweist, wobei die Führungsausnehmung (67, 77) mindestansens gekrümmte Kante (81) hat, die einen Kreisbogen um die Schwenkachse (61, 71) des Schwenkzapfens (65, 75) bildet

2. Chirurgische Greifzange gemäß Anspruch 1,
**dadurch gekennzeichnet, dass** die andere der Greifbacken (70) nicht beweglich im Grundkörper (10) eingesetzt ist.

3. Chirurgische Greifzange gemäß Anspruch 1,
**dadurch gekennzeichnet,**
- **dass** gegenüber dem Grundkörper (10) beide Greifbacken (60, 70) beweglich angeordnet sind, wobei jede Greifbacke (60, 70) eine gegenüber dem Grundkörper (10) ortsfeste Schwenkachse und einen Hebelarm (64, 74) hat und wobei die Hebelarme (64, 74) über mindestens ein Schubelement (40) angelenkt sind,
- **dass** jede Greifbacke (60, 70) eine eigene Schwenkachse (61, 71) hat und
- **dass** die einzelne Schwenkachse (61, 71) von der Mittellinie (13) des Grundkörpers (10) mindestens einen Abstand (21) hat, der größer als 38% der maximalen Grundkörperbreite oder des maximalen Grundkörperdurchmessers (22) ist.

4. Chirurgische Greifzange gemäß einem vorstehenden Anspruch,
**dadurch gekennzeichnet, dass** im Grundkörper (10) ein Schubelement (40) die eine bewegliche Greifbacke (60) bzw. die beiden beweglichen Greifbacken (60, 70) anlenkt.

5. Chirurgische Greifzange gemäß einem vorstehenden Anspruch,
**dadurch gekennzeichnet, dass** das Schubelement (40) im Grundkörper (10) mindestens zweifach geführt ist.

6. Chirurgische Greifzange gemäß Anspruch 3,
**dadurch gekennzeichnet, dass** die Schwenkachsen (61, 71) der beiden beweglichen Greifbacken (60, 70) parallel zueinander angeordnet sind.

7. Chirurgische Greifzange gemäß einem vorstehenden Anspruch,
**dadurch gekennzeichnet, dass** die Schwenkachse (61) der einen beweglichen Greifbacke (60) bzw. die Schwenkachsen (61, 71) der beiden beweglichen Greifbacken (60, 70) in einer Ebene liegen, die normal zur Mittellinie (13) des Grundkörpers (10) orientiert ist.

8. Chirurgische Greifzange nach einem der Ansprüche 1 bis 7,
**dadurch gekennzeichnet, dass** die grundkörperseitige Ausnehmung (17, 27) den greifbackenseitigen Schwenkzapfen (65, 75) nur teilweise umschließt.

9. Chirurgische Greifzange nach einem der Ansprüche 1 bis 8,
**dadurch gekennzeichnet, dass** sich die Kante (81) der Führungsausnehmung (67, 77) an einem im Grundkörper (10) gelagerten Führungsbolzen (30) abstützt.

10. Chirurgische Greifzange gemäß einem vorstehenden Anspruch,
**dadurch gekennzeichnet, dass der** Grundkörper (10) und das Schubelement (40) getriebetechnisch ein Schiebepaar bilden, wobei das Schubelement (40) im Grundkörper (10) verdrehsicher gelagert ist.

11. Chirurgische Greifzange gemäß einem vorstehenden Anspruch,
**dadurch gekennzeichnet, dass** der Grundkörper (10), das Schubelement (40) und die Greifbacken (60, 70) getriebetechnisch eine bzw. zwei schwingende Kurbelschleifen bilden, wobei die einzelne Kulissenausnehmung (67, 77) der Kurbelschleifen jeweils an einem Hebelarm (64, 74) der jeweiligen beweglichen der Greifbacken (60, 70) angeordnet ist.

## Claims

1. Surgical tongs with two tong jaws (60, 70; 60a, 70a), wherein at least one of the tong jaws (60) is arranged mobile in relation to a base body (10), wherein the mobile tong jaw (60; 60a) comprises a swivel axis stationary in relation to the base body (10) and a lever arm (64) and wherein the lever arm (64) is hinged via at least one thrust element (40), wherein the mobile tong jaw (60) has its own swivel axis (61; 62), wherein the swivel axis (61) has at least a distance (21) from the centre line (13) of the base body (10), which distance is greater than 38% of the maximum base body width or maximum base body diameter (22),
**characterised in that**
- the swivel axis (61) of the one mobile tong jaw (60) is formed by a pivot pin (65, 75) on the tong jaw side and a recess (17, 27) on the base body side, and
- the individual mobile or both mobile tong jaw(s) (60, 70) in the vicinity of the swivel pin (65, 75) comprises a guide recess (67, 77), the guide recess (67, 77) having at least one curved edge (81) which forms a circle arc about the swivel axis (61, 71) of the swivel pin (65, 75).

2. Surgical tongs according to claim 1, **characterised in that** the other of the tong jaws (70) is inserted immobile in the base body (10).

3. Surgical tongs according to claim 1, **characterised in that**
- both tong jaws (60, 70) are arranged mobile in relation to the base body (10), wherein each tong jaw (60, 70) comprises a swivel axis stationary in relation to the base body (10) and a lever arm (64, 74) and wherein the lever arms (64, 74) are hinged via at least one thrust element (40),
- each tong jaw (60, 70) has its own swivel axis (61, 71), and
- the individual swivel axis (61, 71) has at least a distance (21) from the centre line (13) of the base body (10), which distance is greater than 38% of the maximum base body width or maximum base body diameter (22).

4. Surgical tongs according to any of the preceding claims, **characterised in that** in the base body a thrust element (40) pivots the one mobile tong jaw (60) or both mobile tong jaws (60, 70).

5. Surgical tongs according to any of the preceding claims, **characterised in that** the thrust element (40) is guided at least twice in the base body (10).

6. Surgical tongs according to claim 3, **characterised in that** the swivel axes (61, 71) of the two mobile tong jaws (60, 70) are arranged parallel to each other.

7. Surgical tongs according to any of the preceding claims, **characterised in that** the swivel axis (61) of the one mobile tong jaw (60) or the swivel axes (61, 71) of both mobile tong jaws (60, 70) lie in a plane oriented perpendicular to the centre line (13) of the base body (10).

8. Surgical tongs according to any of claims 1 to 7, **characterised in that** the body-side recess (17, 27) only partly surrounds the swivel pin (65, 75) on the tong jaw side.

9. Surgical tongs according to any of claims 1 to 8, **characterised in that** the edge (81) of the guide recess (67, 77) rests on a guide bolt (30) mounted in the base body (10).

10. Surgical tongs according to any of the preceding claims, **characterised in that** the base body (10) and the thrust element (40) form as a mechanism a transfer pair, wherein the thrust element (40) is mounted twist-rigid in the base body (10).

11. Surgical tongs according to any of the preceding claims, **characterised in that** the base body (10), the thrust element (40) and the tong jaws (60, 70) form as mechanisms one or two slider cranks, wherein the individual slider recesses (67, 77) of the slider cranks are each arranged on a lever arm (64, 75) of the respective mobile one of the tong jaws (60, 70).

## Revendications

1. Pince chirurgicale comportant deux mors (60, 70 ; 60a, 70a),
* au moins l'un des mors (60) étant mobile par rapport à un corps de base (10),
* celui des mors (60, 60a) qui est mobile par rapport au corps de base (10) a un axe de pivotement fixe et un bras de levier (64), et
* le bras de levier (64) est articulé par au moins un élément de coulissement (40),
* le mors mobile (60) a son propre axe de pivotement (61, 62),
* l'axe de pivotement (61) est écarté de l'axe géométrique (13) du corps de base (10) d'au moins une distance (21) supérieure à 38 % de la largeur maximale du corps de base ou du diamètre maximum du corps de base (22),
**caractérisée en ce que**
- l'axe de pivotement (61) du mors mobile (60) est formé par un goujon de pivotement (65, 75) du côté du mors et par une cavité (17, 27) du côté du corps de base, et
- l'unique mors mobile ou les deux mors mobiles (60, 70) comportent à proximité du goujon de pivotement (65, 75), une cavité de guidage (67, 77), et
* la cavité de guidage (67, 77) ayant au moins un bord courbe (81) en are de cercle centré sur l'axe de pivotement (61, 71) du goujon de pivotement (65, 75).

2. Pince chirurgicale selon la revendication 1,
**caractérisée en ce que**
l'autre mors (70) est monté de manière non mobile dans le corps de base (10),

3. Pince chirurgicale selon la revendication 1,
**caractérisée en ce que**
- les deux mors (60, 70) sont montés mobiles, par rapport au corps de base (10),
* chacun des mors (60, 70) a un axe de pivotement fixe par rapport au corps de base (10) et un bras de levier (64, 74), et
* les bras de levier (64, 74) sont articulés par l'intermédiaire d'au moins un élément de coulissement (40),
- chaque mors (60, 70) a son propre axe de pivotement (61, 71), et
- les axes de pivotement séparés (61, 71) sont écartés par rapport à l'axe géométrique (13) du corps de base (10) d'au moins une distance (21) supérieure à 38 % de la largeur maximale du corps de base ou du diamètre maximum du corps de base (22).

4. Pince chirurgicale selon l'une des revendications précédentes,
**caractérisée en ce que**
le corps de base (10) comporte un élément de coulissement (40) qui est articulé sur le mors mobile (60) ou les deux mors mobiles (60, 70).

5. Pince chirurgicale selon l'une des revendications précédentes,
**caractérisée en ce que**
l'élément de coulissement (40) est au moins dédoublé dans le corps de base (10).

6. Pince chirurgicale selon l'une revendication 3,
**caractérisée en ce que**
les axes de pivotement (61, 71) des deux mors mobiles (60, 70) sont parallèles.

7. Pince chirurgicale selon l'une des revendications précédentes,
**caractérisée en ce que**
l'axe de pivotement (61) de l'un (60) des mors mobiles ou les axes de pivotement (61, 71) des deux mors mobiles (60, 70) sont situés dans un plan perpendiculaire à l'axe géométrique (13) du corps de base (10).

8. Pince chirurgicale selon l'une des revendications 1 à 7,
**caractérisée en ce que**
**en ce que** la cavité (17, 27) du côté du corps de base n'entoure que partiellement les goujons de pivotement (65, 75) des mors.

9. Pince chirurgicale selon l'une des revendications 1 à 8,
**caractérisée en ce que**
le bord (81) de la cavité de guidage (67, 77) s'appuie sur un goujon de guidage (30) logé dans le corps de base (10).

10. Pince chirurgicale selon l'une des revendications précédentes,
**caractérisée en ce que**
le corps de base (10) et l'élément de coulissement (40) constituent une paire de coulissement au sens de la technique de transmission,
* l'élément coulissant (40) étant monté de manière bloquée en rotation dans le corps de base (10).

11. Pince chirurgicale selon l'une des revendications précédentes,
**caractérisée en ce que**
le corps de base (10), l'élément coulissant (40) et les mors (60, 70) constituent, sur le plan de la technique de transmission, une boucle à manivelle flottante, les cavités de guidage, séparées (67, 77) des boucles à manivelle étant installées respectivement sur le bras de levier (64, 74) du mors mobile (60, 70) respectif.
